# NOUVEAU FASCICULE DE BREVET EUROPEEN

(11) **EP 2 062 615 B2**
(45) Date de publication et mention de la décision concernant l'opposition: **06.03.2019**
(45) Mention de la délivrance du brevet: 06.01.2016
(21) Numéro de dépôt: 08168553.9
(22) Date de dépôt: 07.11.2008
(51) Int. Cl.: A61Q 5/10, A61K 8/73, A61K 8/42, A61K 8/36, A61K 8/37

(54) **Composition comprenant un dérivé de cellulose modifié, un ester d'acide gras et des colorants d'oxydation, procédé de teinture d'oxydation et utilisation**
Zusammensetzung, die ein modifiziertes Zellulosederivat, einen Fettsäureester und Oxydationsfarbstoffe umfasst, Oxydationsfärbe- und Anwendungsverfahren
Composition comprising a modified cellulose derivative, a fatty acid ester and oxidising dyes, dyeing method for oxidation and use.

(30) Priorité: 09.11.2007 FR 0758913
(43) Date de publication de la demande: 27.05.2009
(60) Demande divisionnaire: 13154932.1
(73) Titulaire: L'Oréal, 75008 Paris (FR)
(72) Inventeur: Audousset, Marie-Pascale, 92600, Asnieres (FR)
(74) Mandataire: Casalonga, Axel

(56) Documents cités:
- EP-A- 1 426 032
- EP-A- 1 707 183
- EP-A- 1 707 190
- EP-A1- 1 142 557
- EP-A1- 1 426 039
- EP-A1- 1 707 182
- WO-A-98/03150
- WO-A-03/053329
- WO-A1-99/17724

## Description

La présente demande a pour objet une composition de teinture d'oxydation des fibres kératiniques, et en particulier des fibres kératiniques humaines telles que les cheveux, comprenant un ou plusieurs dérivé(s) non ionique(s) de cellulose modifié(s) par un ou plusieurs groupement(s) hydrophobe(s) particulier(s), un ou plusieurs ester(s) d'acide gras particulier(s), et un ou plusieurs colorant(s) d'oxydation.

L'invention a aussi pour objet l'utilisation de cette composition pour la teinture des fibres kératiniques ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho-ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences.

Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui comporte en général des zones différemment sensibilisées (i.e. abîmées) de sa pointe à sa racine.

Par ailleurs, les compositions obtenues doivent, en outre, présenter de bonnes propriétés rhéologiques, tout en conservant de bonnes propriétés de coloration. En particulier, ces compositions ne doivent pas couler sur le visage ou en dehors des zones que l'on se propose de teindre, lors de leur application, notamment après mélange avec un agent oxydant.

Il est connu du document EP-1 707 190 d'utiliser des compositions colorantes particulières comprenant un tensioactif non-ionique et/ou un tensioactif anionique, une cellulose non ionique modifiée par des groupements comportant au moins une chaîne hydrocarbonée en C6-C30, au moins un polymère associatif cationique et au moins 40 % en poids d'eau. Il est également connu du document EP-A-1 707 183 des compositions colorantes particulières comprenant un alcool gras, un ester d'acide gras et d'alcool en C1-C10, un tensioactif non ionique et/ou un tensioactif anionique, un polymère associatif non ionique et au moins 55 % en poids d'eau.

Il est déjà connu de la demande WO 98/03150 d'améliorer la puissance de la coloration en associant une base d'oxydation para-phénylènediamine et au moins un polymère amphiphile non ionique du type hydroxycellulose modifiée par un groupement hydrophobe.

Cependant, ces compositions ne satisfont pas pleinement les exigences précitées et peuvent être améliorées, notamment en termes de propriétés tinctoriales, en particulier au niveau de la sélectivité et de la puissance de coloration. Le but de la présente invention est l'obtention de compositions de coloration capillaire stables, notamment sous forme de crèmes, faciles à préparer et à appliquer, pouvant contenir des concentrations élevées de colorants sous forme de sels, ayant de bonnes qualités rhéologiques et conduisant à des colorations intenses, peu sélectives et résistantes aux diverses agressions que peuvent subir les fibres kératiniques.

Ce but est atteint par la présente invention qui a pour objet une composition tinctoriale pour fibres kératiniques, et en particulier pour fibres kératiniques humaines telles que les cheveux, comprenant, dans un milieu approprié pour la teinture :
(A) un ou plusieurs dérivé(s) non ionique(s) de cellulose comprenant un ou plusieurs substituant(s) hydrophobe(s) comprenant de 8 à 30 atomes de carbone ;
(B) un ou plusieurs ester(s) d'acide gras en C₈-C₃₀, tels que défini en revendication 1; et
(C) un ou plusieurs colorant(s) d'oxydation.

Les compositions tinctoriales selon l'invention présentent notamment les propriétés suivantes :
- elles permettent d'obtenir des compositions de viscosité correspondant à une crème qui sont stables dans le temps,
- elles se distinguent par une facilité de mélange avec la composition oxydante,
- elles se distinguent par les qualités rhéologiques des crèmes obtenues (bonne viscosité de crème en mélange),
- elles sont faciles à appliquer après mélange avec la composition oxydante au moment de la mise en oeuvre de la coloration (qualités d'usage sur tête).

En outre, les compositions selon l'invention permettent l'obtention de compositions capables de conduire à des colorations aux nuances variées, chromatiques, puissantes, esthétiques, peu sélectives, uniformes sur l'ensemble des fibres kératiniques et en particulier des fibres kératiniques humaines telles que les cheveux, et résistant bien aux diverses agressions que peuvent subir les fibres.

Un autre objet de la présente invention consiste en un procédé de teinture des fibres kératiniques dans lequel la composition cosmétique selon l'invention est mise en oeuvre.

Un troisième objet de l'invention concerne l'utilisation de cette composition cosmétique pour la teinture des fibres kératiniques, et en particulier des fibres kératiniques humaines, telles que les cheveux.

D'autres caractéristiques, aspects, objets et avantages de la présente invention apparaîtront encore plus clairement à la lecture de la description et des exemples qui suivent.

A moins d'une indication différente, les bornes des gammes de valeurs qui sont données dans le cadre de la présente invention sont incluses dans ces gammes.

Par « dérivé(s) de cellulose », on entend un (ou des) composé(s) comportant au moins un motif cellobiose de structure suivante : 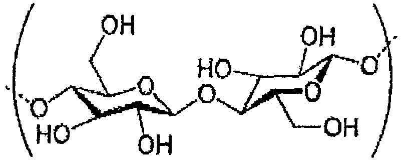 dans laquelle, un ou plusieurs groupe(s) hydroxyle peut (ou peuvent) être substitué(s).

Le ou les dérivés non ioniques de cellulose à substituant(s) hydrophobe(s) (A) conformes à la présente invention, sont des polymères amphiphiles présentant un caractère associatif. En effet, ils comprennent des motifs hydrophiles et des motifs hydrophobes et sont capables d'interagir et de s'associer entre eux ou à d'autres molécules, de manière réversible, en particulier, grâce à la présence de leurs chaînes hydrophobes.

De préférence, le dérivé de cellulose de l'invention est un éther de cellulose comprenant un ou plusieurs substituant(s) hydrophobe(s) comprenant de 8 à 30 atomes de carbone.

Le ou les dérivés non ioniques de cellulose à substituant(s) hydrophobe(s) conformes à la présente invention, sont préparés généralement à partir d'éthers non-ioniques de cellulose hydrosolubles, dont on substitue tout ou partie des fonctions hydroxyle réactives par une ou plusieurs chaîne(s) hydrophobe(s) comprenant de 8 à 30 atomes de carbone, de préférence de 10 à 22 atomes de carbone, et mieux encore 16 atomes de carbone. Les étapes de réactions en jeu dans la préparation des dérivés de cellulose de l'invention sont connues de l'homme du métier.

Les éthers non-ioniques de cellulose choisis pour préparer les dérivés non-ioniques de cellulose à substituant(s) hydrophobe(s) selon l'invention, ont de préférence un degré de substitution non-ionique, par exemple en groupement(s) méthyle, hydroxyéthyle ou hydroxypropyle, suffisant pour être hydrosolubles, c'est-à-dire former une solution sensiblement limpide lorsqu'ils sont dissous dans l'eau à 25 °C à la concentration de 1 % en poids.

Les éthers non ioniques de cellulose choisis pour préparer les dérivés non ioniques de cellulose à substituant(s) hydrophobe(s) selon l'invention, possèdent préférentiellement une masse molaire moyenne en nombre relativement faible, inférieure à 800 000 g/mole, de préférence allant de 50 000 et 700 000 g/mole et de manière plus préférée allant de 200 000 à 600 000 g/mole.

De préférence, le dérivé de cellulose de l'invention est une hydroxyéthylcellulose comprenant un ou plusieurs substituant(s) hydrophobe(s) comprenant de 8 à 30 atomes de carbone.

Les dérivés non-ioniques de cellulose utilisés selon l'invention sont substitués , par une ou plusieurs chaîne(s) hydrocarbonée(s) en C₈-C₃₀ linéaires, ramifiées ou cycliques, saturées ou insaturées, aliphatiques ou aromatiques, pouvant être rattachées au substrat éther de cellulose par une liaison éther, ester, ou uréthane, de préférence éther.

Selon un mode de réalisation, le ou les substituants hydrophobes utilisés comme substituants des dérivés non-ioniques de cellulose selon la présente invention sont des groupes alkyle, arylalkyle ou alkylaryle en C₈-C₃₀, de préférence en C₁₀-C₂₂.

De préférence, le ou les substituants hydrophobes selon la présente invention sont des chaînes alkyles saturées.

Selon un mode de réalisation préféré, le ou les substituants hydrophobes selon la présente invention sont des groupements cétyle.

Les dérivés non-ioniques de cellulose à substituant(s) hydrophobe(s) selon l'invention, présentent une viscosité de préférence comprise entre 100 et 100 000 mPa.s, et de préférence entre 200 et 20 000 mPa.s, mesurée à 25 °C dans une solution à 1 % en poids de polymère dans l'eau, cette viscosité étant déterminée de manière conventionnelle à l'aide d'un viscosimètre de type Brookfield LVT à 6 tours par minute avec le mobile n° 3.

Le degré de substitution hydrophobe des dérivés non-ioniques de cellulose hydrophiles utilisés selon l'invention, va préférentiellement de 0,1 à 10 % en poids, plus préférentiellement de 0,1 à 1 % en poids et de manière particulièrement préférée de 0,4 à 0,8 % en poids, du poids total du polymère.

Parmi les dérivés non-ioniques de cellulose à substituant(s) hydrophobe(s) utilisables dans les compositions de l'invention, on peut citer, de préférence, les cétyl hydroxyéthylcelluloses commercialisées sous les dénominations Natrosol Plus Grade 330 CS et Polysurf 67 CS (INCI : Cetyl Hydroxyethylcellulose) par la société Aqualon/Hercules.

La concentration en dérivé(s) non-ionique(s) de cellulose à substituant(s) hydrophobe(s) (A) des compositions selon l'invention va de préférence de 0,01 à 10 % en poids, en particulier de 0,05 à 3 % en poids, et de manière plus préférée de 0,1 à 1 % en poids, par rapport au poids total de la composition.

Le ou les ester(s) d'acide gras (B) utilisable(s) selon l'invention, sont des mono- ou polyesters, choisis parmi les monoesters, diesters et triesters issus de la réaction de monoacides ou diacides, linéaires ou ramifiés, saturés ou insaturés, comportant de 8 à 30 atomes de carbone, éventuellement hydroxylés, avec des des polyols, saturés ou insaturés, linéaires, ramifiés ou cycliques, comportant de 2 à 1000 atomes de carbone et de 1 à 30 groupement(s) hydroxyle, les polyols considérés étant choisis parmi l'éthylène glycol, le propylene glycol, le glycérol, les polyéthylène glycols et les polypropylène glycols, les esters étant choisis parmi, les mono- ou distéarate d'éthylène glycol, les mono- ou distéarate de polyéthylène glycols tels que le PEG-40 stéarate, l'isostéarate de glycéryle, le dipélargonate de propylèneglycol, le trihydroxystéarate de glycéryle.

Les polyols, qui ne sont pas des polyéthylèneglycols et/ou des polypropylèneglycols peuvent être éventuellement polyoxyalkylénés, et plus particulièrement polyoxyéthylénés et/ou polyoxypropylénés, le nombre de moles d'oxyde d'éthylène et/ou d'oxyde de propylène par mole d'ester étant alors de préférence compris entre 2 et 400, mieux entre 2 et 200.

De préférence, les polyols, s'ils sont différents des polyéthylèneglycols et/ou des polypropylèneglycols, ne sont pas polyoxyalkylénés.

Le ou les ester(s) d'acide gras utilisable(s) selon l'invention sont généralement non ioniques, c'est-à-dire qu'ils ne comportent pas de charges ioniques.

De préférence, l'alcool de l'ester comporte plus de 3 atomes de carbone.

La concentration en ester(s) d'acide gras (B) des compositions selon l'invention va de préférence de 0,01 à 10 % en poids, en particulier de 0,2 à 10 % en poids, et de manière plus préférée de 0,5 à 6 % en poids, par rapport au poids total de la composition.

La composition selon l'invention peut comprendre en outre un ou plusieurs amides d'acide gras. Le ou les amides d'acide gras utilisable(s) selon l'invention sont des amides issus de la réaction d'une alcanolamine et d'un acide gras en C₁₄-C₃₀. Ils sont de préférence choisis parmi les amides d'une alcanolamine en C₂-C₁₀ et d'un acide gras en C₁₄-C₃₀, et encore plus préférentiellement parmi les amides d'une alcanolamine en C₂-C₆ et d'un acide gras en C₁₄-C₂₂.

L'alcanolamine peut-être une mono- ou une dialcanolamine. L'acide gras peut être saturé ou insaturé, linéaire ou ramifié.

Le ou les amides d'acide gras utilisables selon l'invention sont généralement non ioniques, c'est-à-dire qu'elles ne comportent pas de charges ioniques.

L'amide d'une alcanolamine et d'un acide gras en C₁₄-C₃₀ est de préférence choisi parmi :
- le diéthanolamide d'acide oléique, tel que l'amide commercialisé sous la dénomination commerciale MEXANYL® GT par la société CHIMEX,
- le monoéthanolamide d'acide myristique, tel que l'amide commercialisé sous la dénomination commerciale COMPERLAN® MM par la société COGNIS,
- le diéthanolamide d'acides gras de soja, tel que l'amide commercialisé sous la dénomination commerciale COMPERLAN® VOD par la société COGNIS,
- l'éthanolamide d'acide stéarique, tel que l'amide commercialisé sous la dénomination commerciale MONAMID® S par la société UNIQEMA,
- le monoisopropanolamide d'acide oléique, tel que l'amide commercialisé sous la dénomination commerciale WITCAMIDE® 61 par la société WITCO,
- le diéthanolamide d'acide linoléique, tel que l'amide commercialisé sous la dénomination commerciale PURTON® SFD par la société ZSCHIMMER SCHWARZ,
- le monoéthanolamide d'acide stéarique, tel que l'amide commercialisé sous la dénomination commerciale MONAMID® 972 par la société ICI/UNIQEMA,
- le monoéthanolamide d'acide béhénique, tel que l'amide commercialisée sous la dénomination commerciale INCROMIDE® BEM de CRODA,
- le monoisopropanolamide d'acide isostéarique, tel que l'amide commercialisé sous la dénomination commerciale WITCAMIDE® SPA par la société WITCO,
- le diéthanolamide d'acide érucique, tel que l'amide commercialisé sous la dénomination commerciale diéthanolamide d'acide érucique par la société STEARINERIES DUBOIS,
- le monoéthanolamide d'acide ricinoléique, tel que l'amide commercialisé sous la dénomination commerciale monoéthanolamide ricinoléique par la société STEARINERIES DUBOIS,

La concentration en amide(s) d'acide gras des compositions selon l'invention va de préférence de 0 à 10 %, en particulier de 0,2 à 10 % en poids, et de manière plus préférée de 0,5 à 6 % en poids, par rapport au poids total de la composition.

Selon un mode de réalisation particulier, les compositions tinctoriales de l'invention comprennent, dans un milieu approprié pour la teinture :
(A) un ou plusieurs dérivé(s) non ionique(s) de cellulose comprenant un ou plusieurs substituant(s) hydrophobe(s) comprenant de 8 à 30 atomes de carbone tel(s) que défini(s) précédemment ;
(B)
   (i) un ou plusieurs amide(s) d'une alcanolamine et d'un acide gras en C₁₄-C₃₀ et
   (ii) un ou plusieurs ester(s) d'acide gras en C₈-C₃₀ ; et
(C) un ou plusieurs colorant(s) d'oxydation, et de préférence au moins une base d'oxydation et au moins un coupleur d'oxydation.

Le (ou les) colorant(s) d'oxydation (C) utilisable(s) selon l'invention est (ou sont) de préférence choisi(s) parmi les bases d'oxydation, les coupleurs d'oxydation, et leurs sels d'addition.

A titre d'exemple, les bases d'oxydation utilisables sont choisies parmi les para-phénylènediamines, les bis-phénylalkylènediamines, les para-aminophénols, les bis-para-aminophénols, les ortho-aminophénols, les bases hétérocycliques et leurs sels d'addition.

Parmi les para-phénylènediamines, on peut citer à titre d'exemple, la para-phénylènediamine, la para-toluylènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino-2-méthyl aniline, la 4-N,N-bis-(β-hydroxyéthyl)amino-2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylène-diamine, la 4-aminophénylpyrrolidine, la 2-thiényl para-phénylènediamine, le 2-β hydroxyéthylamino-5-aminotoluène, la 3-hydroxy-1-(4'-aminophényl)pyrrolidine et leurs sels d'addition avec un acide.

Parmi les para-phénylènediamines citées ci-dessus, la para-phénylènediamine, la para-toluylènediamine, la 2-isopropyl para-phénylènediamine, la 2-β-hydroxyéthyl para-phénylènediamine, la 2-β-hydroxyéthyloxy para-phénylène-diamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino-3-méthyl phénol, le 4-amino-3-fluoro phénol, le 4-amino-3-hydroxyméthyl phénol, le 4-amino-2-méthyl phénol, le 4-amino-2-hydroxyméthyl phénol, le 4-amino-2-méthoxyméthyl phénol, le 4-amino-2-aminométhyl phénol, le 4-amino-2-(β-hydroxyéthyl aminométhyl)phénol, le 4-amino 2-fluoro phénol, et leurs sels d'addition avec un acide.

Parmi les ortho-aminophénols, on peut citer à titre d'exemple, le 2-aminophénol, le 2-amino-5-méthylphénol, le 2-amino-6-méthylphénol, le 5-acétamido-2-aminophénol, et leurs sels d'addition avec un acide.

Parmi les bases hétérocycliques, on peut citer à titre d'exemple, les dérivés pyridiniques, les dérivés pyrimidiniques, les dérivés pyrazoliques, les dérivés de pyrazolone, et leurs sels d'addition.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N7, N7-tétraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques utilisables, on peut citer par exemple les composés décrits dans les brevets DE-A-38 43 892, DE-A-41 33 957 et demandes de brevet WO 94/08969, WO 94/08970, FR-A-2 733 749 et DE-A-195 43 988 comme le 4,5-diamino-1-méthylpyrazole, le 4,5-diamino-1-(β-hydroxyéthyl)pyrazole, le 3,4-diaminopyrazole, le 4,5-diamino-1-(4'-chlorobenzyl)pyrazole, le 4,5-diamino-1,3-diméthylpyrazole, le 4,5-diamino-3-méthyl-1-phényl-pyrazole, le 4,5-diamino-1-méthyl-3-phénylpyrazole, le 4-amino-1,3-diméthyl-5-hydrazinopyrazole, le 1-benzyl-4,5-diamino-3-méthylpyrazole, le 4,5-diamino-3-tert-butyl-1-méthylpyrazole, le 4,5-diamino-1-tert-butyl-3-méthylpyrazole, le 4,5-diamino-1-(β-hydroxyéthyl)-3-méthylpyrazole, le 4,5-diamino-1-éthyl-3-méthylpyrazole, le 4,5-diamino-1-éthyl-3-(4'-méthoxyphényl)pyrazole, le 4,5-diamino-1-éthyl-3-hydroxyméthylpyrazole, le 4,5-diamino-3-hydroxyméthyl-1-méthylpyrazole, le 4,5-diamino-3-hydroxyméthyl-1-isopropylpyrazole, le 4,5-diamino-3-méthyl-1-isopropylpyrazole, le 4-amino-5-(2'-aminoéthyl)amino-1,3-diméthylpyrazole, le 3,4,5-triaminopyrazole, le 1-méthyl-3,4,5-triaminopyrazole, le 3,5-diamino-1-méthyl-4-méthylaminopyrazole, le 3,5-diamino-4-(β-hydroxyéthyl)amino-1-méthylpyrazole, et leurs sels d'addition.

Parmi les dérivés de pyrazolone utilisables, on peut citer par exemple les composés suivants et leurs sels d'addition :
2,3-diaminodihydropyrazolone ;
4,5-diamino-1,2-diméthyl-1,2-dihydropyrazol-3-one ;
4-amino-5-méthylamino-1,2-diméthyl-1,2-dihydropyrazol-3-one ;
4-amino-5-diméthylamino-1,2-diméthyl-1,2-dihydropyrazol-3-one ;
4-amino-5-(2-hydroxyéthyl)amino-1,2-diméthyl-1,2-dihydropyrazol-3-one ;
4-amino-5-(pyrrolidin-1-yl)-1,2-diméthyl-1,2-dihydropyrazol-3-one ;
4-amino-5-(pipéridin-1-yl)-1,2-diméthyl-1,2-dihydropyrazol-3-one ;
4,5-diamino-1,2-di-(2-hydroxyéthyl)-1,2-dihydropyrazol-3-one ;
4-amino-5-méthylamino-1,2-di-(2-hydroxyéthyl)-1,2-dihydropyrazol-3-one ;
4-amino-5-diméthylamino-1,2-di-(2-hydroxyéthyl)-1,2-dihydropyrazol-3-one ;
4-amino-5-(2-hydroxyéthyl)amino-1,2-di-(2-hydroxyéthyl)-1,2-dihydropyrazol-3-one ;
4-amino-5-(pyrrolidin-1-yl)-1,2-di-(2-hydroxyéthyl)-1,2-dihydropyrazol-3-one ;
4-amino-5-(pipéridin-1-yl)-1,2-di-(2-hydroxyéthyl)-1,2-dihydropyrazol-3-one ;
4,5-diamino-1,2-diéthyl,-1,2-dihydropyrazol-3-one ;
4,5-diamino-1,2-diphényl-1,2-dihydropyrazol-3-one ;
4,5-diamino-1-éthyl-2-méthyl-1,2-dihydropyrazol-3-one ;
4,5-diamino-2-éthyl-1-méthyl-1,2-dihydropyrazol-3-one ;
4,5-diamino-1-phényl-2-méthyl-1,2-dihydropyrazol-3-one ;
4,5-diamino-2-phényl-1-méthyl-1,2-dihydropyrazol-3-one ;
4,5-diamino-1-(2-hydroxyéthyl)-2-méthyl-1,2-dihydropyrazol-3-one ;
4,5-diamino-2-(2-hydroxyéthyl)-1-méthyl-1,2-dihydropyrazol-3-one ;
2,3-diamino-6,7-dihydro-1H,5H-pyrazolo[1,2-a]pyrazol-1-one ;
2-amino-3-méthylamino-6,7-dihydro-1H,5H-pyrazolo[1,2-a]pyrazol-1-one ;
2-amino-3-diméthylamino-6,7-dihydro-1H,5H-pyrazolo[1,2-a]pyrazol-1-one ;
2-amino-3-éthylamino-6,7-dihydro-1H,5H-pyrazolo[1,2-a]pyrazol-1-one ;
2-amino-3-isopropylamino-6,7-dihydro-1H,5H-pyrazolo[1,2-a]pyrazol-1-one ;
2-amino-3-(2-hydroxyéthyl)amino-6,7-dihydro-1H,5H-pyrazolo[1,2-a]-pyrazol-1-one ;
2-amino-3-(2-hydroxypropyl)amino-6,7-dihydro-1H,5H-pyrazolo[1,2-a]-pyrazol-1-one ;
2-amino-3-bis(2-hydroxyéthyl)amino-6,7-dihydro-1H,5H-pyrazolo[1,2-a]-pyrazol-1-one ;
2-amino-3-(pyrrolidin-1-yl)-6,7-dihydro-1H,5H-pyrazolo[1,2-a]pyrazol-1-one ;
2-amino-3-(3-hydroxy-pyrrolidin-1-yl)-6,7-dihydro-1H,5H-pyrazolo[1,2-a]-pyrazol-1-one ;
2-amino-3-(pipéridin-1-yl)-6,7-dihydro-1H,5H-pyrazolo[1,2-a]pyrazol-1-one ;
2,3-diamino-6-hydroxy-6,7-dihydro-1H,5H-pyrazolo[1,2-a]pyrazol-1-one ;
2,3-diamino-6-méthyl-6,7-dihydro-1H,5H-pyrazolo[1,2-a]pyrazol-1-one ;
2,3-diamino-6,6-diméthyl-6,7-dihydro-1H,5H-pyrazolo[1,2-a]pyrazol-1-one ;
2,3-diamino-5,6,7,8-tétrahydro-1H,6H-pyridazino[1,2-a]pyrazol-1-one ;
2,3-diamino-5,8-dihydro-1H,6H-pyridazino[1,2-a]pyrazol-1-one ;
4-amino-5-diméthylamino-1,2-diéthyl-1,2-dihydropyrazol-3-one ;
4-amino-1,2-diéthyl-5-éthylamino-1,2-dihydropyrazol-3-one ;
4- amino -1,2-diéthyl-5-isopropylamino-1,2-dihydropyrazol-3-one ;
4- amino -1,2-diéthyl-5-(2-hydroxyéthylamino)-1,2-dihydropyrazol-3-one ;
4-amino-5-(2-diméthylaminoéthylamino)-1,2-diéthyl-1,2-dihydropyrazol-3-one ;
4-amino-5-[bis(2-hydroxyéthyl)amino]-1,2-diéthyl-1,2-dihydropyrazol-3-one ;
4-amino-1,2-diéthyl-5-(3-imidazol-1-yl-propylamino)-1,2-dihydropyrazol-3-one ;
4-amino-1.2-diéthyl-5-(3-hydroxypyrrolidin-1-yl)-1,2-dihydropyrazol-3-one ;
4-amino-5-pyrrolidin-1-yl-1,2-diéthyl-1,2-dihydropyrazol-3-one ;
4-amino-5-(3-diméthylaminopyrrolidin-1-yl)-1,2-diéthyl-1,2-dihydropyrazol-3-one ;
4-amino-1,2-diéthyl-5-(4-méthylpipérazin-1-yl)pyrazolidin-3-one.

La concentration en base(s) d'oxydation va en général de 0,001 à 20 % en poids, de préférence de 0,005 à 10 % en poids, et encore plus préférentiellement de 0,01 à 5 % en poids, par rapport au poids total de la composition.

Le (ou les) coupleur(s) d'oxydation présent(s) dans les compositions de l'invention, peut (ou peuvent) être choisi(s) parmi les coupleurs benzéniques, les coupleurs hétérocycliques, les coupleurs naphtaléniques, et leurs sels d'addition.

A titre de coupleurs benzéniques utilisables dans les compositions selon l'invention, on peut citer les méta-aminophénols, les méta-phénylènediamines, les méta-diphénols, ainsi que leurs sels d'addition.

Parmi les coupleurs préférés, on peut citer le 2-méthyl-5-aminophénol, le 5-N-(β-hydroxyéthyl)amino-2-méthyl phénol, le 6-chloro-2-méthyl-5-aminophénol, le 3-aminophénol, le 1,3-dihydroxy benzène, le 1,3-dihydroxy-2-méthyl benzène, le 4-chloro-1,3-dihydroxy benzène, le 2,4-diamino-1-(β-hydroxyéthyloxy)benzène, le 2-amino-4-(β-hydroxyéthylamino)-1-méthoxybenzène, le 1,3-diamino benzène, le 1,3-bis-(2,4-diaminophénoxy)propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(B-hydroxyéthyl)amino-3,4-méthylène dioxybenzène, le 2,6-bis-(β-hydroxyéthylamino)toluène et leurs sels d'addition avec un acide.

La concentration en coupleur(s) d'oxydation va en général de 0,001 à 20 % en poids, de préférence de 0,005 à 10 % en poids, et encore plus préférentiellement de 0,01 à 5 % en poids, par rapport au poids total de la composition.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut, en outre, contenir un ou plusieurs colorant(s) direct(s) pouvant notamment être choisi(s) parmi les colorants nitrés benzéniques, les colorants directs azoïques, les colorants directs méthiniques, les colorants anthraquinoniques, les colorants xanthéniques, les colorants triarylméthaniques et leurs sels d'addition. Ces colorants directs peuvent être de nature non ionique, anionique ou cationique.

Le milieu utilisé dans les compositions selon la présente invention est un milieu aqueux ou un milieu contenant de l'eau et au moins un solvant organique.

Le (ou les) solvant(s) organique(s) utilisé(s) dans les compositions selon la présente invention peut (ou peuvent) être choisi(s) parmi les alcools monohydroxylés et les polyols.

A titre d'alcools monohydroxylés utilisables, on peut citer les alcools inférieurs en C₁-C₄ comme l'éthanol, l'isopropanol, le tertiobutanol, le n-butanol, et leurs mélanges. De préférence, l'alcool utilisé est l'éthanol.

À titre de polyols utilisables, on peut citer le propylèneglycol, les polyéthylèneglycols, la glycérine. A titre de solvants organiques, on peut aussi citer les éthers de polyols comme le 2-butoxyéthanol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

La concentration en solvant(s) organique(s) dans les compositions selon la présente invention est comprise de préférence entre 0 et 30 %, et de manière plus préférée entre 0 et 20 % en poids par rapport au poids total de la composition.

Les compositions selon la présente demande peuvent également contenir, un ou plusieurs agent(s) épaississant(s) encore appelé "agent(s) d'ajustement de la rhéologie" différents des dérivés non-ioniques de cellulose à substituant(s) hydrophobe(s) de l'invention.

L'agent (ou les agents) d'ajustement de la rhéologie peut (ou peuvent) être choisi(s) parmi les agents épaississants minéraux ou organiques, et en particulier les épaississants associatifs polymériques, les alcools gras (alcool oléïque), les dérivés cellulosiques autres que les dérivés de cellulose non-ioniques à substituant(s) hydrophobe(s) (A) selon l'invention (hydroxyéthylcellulose, hydroxypropylcellulose, carboxyméthylcellulose) et les gommes d'origine microbienne (gomme de xanthane, gomme de scléroglucane).

L'agent (ou les agents) d'ajustement de la rhéologie préféré(s) est (ou sont) choisi(s) parmi les alcools gras notamment en C₂₀-C₂₂ et les dérivés de celluloses, autre que les dérivés de cellulose non-ioniques à substituant(s) hydrophobe(s) (A) selon l'invention.

La concentration en agent(s) épaississant(s) est comprise de préférence entre 0,01 et 20 % en poids, et de manière plus préférée entre 1 et 10 % en poids, par rapport au poids total de la composition

La composition tinctoriale conforme à l'invention peut également contenir un ou plusieurs adjuvant(s) utilisé(s) classiquement dans les compositions pour la teinture des cheveux.

Par « adjuvant(s) », on entend un (ou des) additif(s), différent(s) des composés précités, tels que les agents tensioactifs anioniques, cationiques, non-ioniques, amphotères, zwittérioniques ou leurs mélanges ; les polymères non-ioniques, amphotères, zwittérioniques, anioniques, cationiques autres que les dérivés de cellulose non-ioniques à substituant(s) hydrophobe(s) (A) selon l'invention, ou leurs mélanges ; les agents de pénétration ; les agents séquestrants ; les parfums ; les tampons ; les agents dispersants ; les agents conditionneurs tels que par exemple des silicones volatiles ou non volatiles, modifiées ou non modifiées ; les agents filmogènes ; les céramides autres que les amides d'acide gras (B) (i) selon l'invention ; les agents conservateurs ; les agents opacifiants ; les vitamines ; les acides aminés ; les oligopeptides ; les peptides ; les protéines hydrolysées ou non, modifiées ou non ; les enzymes ; les acides et alcools gras ramifiés ou non ; les cires animales, végétales ou minérales ; les acides organiques hydroxylés ; les filtres UV ; les agents anti-oxydants et les agents anti-radicaux libres ; les agents antipelliculaires ; les agents régulateurs de séborrhée ; les agents apaisants ; les huiles minérales ; les polyisobutènes et poly(α-oléfines) ; les pigments ; les acides, bases, plastifiants, charges minérales, nacres, paillettes ; les agents anti-statiques et les agents réducteurs.

Le (ou les) adjuvant(s) ci-dessus est (ou sont), en général, présent(s) en quantité comprise, pour chacun d'eux, de préférence entre 0,01 et 40 % en poids, et de manière plus préférée entre 0,1 et 25 % en poids par rapport au poids de la composition.

Bien entendu, l'homme de l'art veillera à choisir ce (ou ces) éventuel(s) composé(s) complémentaire(s) de manière telle que les propriétés avantageuses attachées intrinsèquement à la composition de teinture d'oxydation conforme à l'invention ne soient pas, ou substantiellement pas, altérées par la (ou les) adjonction(s) envisagée(s).

Le pH de la composition tinctoriale conforme à l'invention va généralement de 3 à 12 environ, et de préférence de 5 à 11 environ. Il peut être ajusté à la valeur désirée au moyen d'agent(s) acidifiant(s) ou alcalinisant(s), habituellement utilisés en teinture des fibres kératiniques ou bien encore à l'aide de système(s) tampon(s) classique(s).

Parmi les agents acidifiants, on peut citer, à titre d'exemple, les acides minéraux ou organiques comme l'acide chlorhydrique, l'acide orthophosphorique, l'acide sulfurique, les acides sulfoniques et les acides carboxyliques comme l'acide acétique, l'acide tartrique, l'acide citrique et l'acide lactique.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (I) suivante : 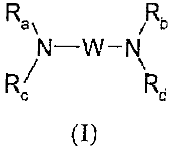 dans laquelle :
▪ W est un reste propylène éventuellement substitué par un groupe hydroxyle ou un groupe alkyle en C₁-C₄ ;
▪ Rₐ, R_{b}, R_{c} et R_{d}, identiques ou différents, représentent un atome d'hydrogène, un groupe alkyle en C₁-C₄ ou hydroxyalkyle en C₁-C₄.

La composition tinctoriale selon l'invention peut se présenter sous des formes diverses, telles que sous forme de crèmes, de gels, ou sous toute autre forme appropriée pour réaliser une teinture des fibres kératiniques, et notamment des cheveux humains.

Le procédé de teinture des fibres kératiniques de la présente invention est un procédé dans lequel on applique sur les fibres la composition selon la présente invention telle que définie précédemment, de préférence en présence d'au moins un agent oxydant, pendant un temps suffisant pour développer la couleur désirée. La couleur peut être révélée à pH acide, neutre ou alcalin et l'agent (ou les agents) oxydant(s) peut (ou peuvent) être ajouté(s) à la composition de l'invention juste au moment de l'emploi ou il(s) peut (ou peuvent) être mis en oeuvre à partir d'une composition oxydante le(s) contenant, appliquée simultanément ou séquentiellement à la composition de l'invention.

Selon un mode de réalisation particulier, la composition selon la présente invention est une composition prête à l'emploi, mélangée, de préférence au moment de l'emploi, à une composition contenant, dans un milieu approprié pour la teinture, au moins un agent oxydant, cet agent (ou ces agents) oxydant(s) étant présent(s) en une quantité suffisante pour développer une coloration. Le mélange obtenu est ensuite appliqué sur les fibres kératiniques. Après un temps de pause de 3 à 50 minutes environ, de préférence, 5 à 30 minutes environ, les fibres kératiniques sont rincées, lavées au shampooing, rincées à nouveau, puis séchées.

Les agents oxydants classiquement utilisés pour la teinture d'oxydation des fibres kératiniques sont, par exemple, le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases, ces oxydoréductases étant éventuellement associées à leurs cofacteurs habituels tels que l'acide urique pour les uricases. L'agent oxydant préféré est le peroxyde d'hydrogène.

La composition oxydante peut également renfermer divers adjuvants utilisés classiquement dans les compositions pour la teinture des cheveux, tels que définis précédemment.

Le pH de la composition oxydante renfermant l'agent oxydant est tel qu'après mélange avec la composition tinctoriale, le pH de la composition résultante appliquée sur les fibres kératiniques varie, de préférence, de 3 à 12 environ, et préférentiellement, de 5 à 10. Il peut être ajusté à la valeur désirée au moyen d'agent(s) acidifiant(s) ou alcalinisant(s) habituellement utilisé(s) en teinture des fibres kératiniques, tel(s) que défini(s) précédemment.

La composition prête à l'emploi qui est finalement appliquée sur les fibres kératiniques, peut se présenter sous des formes diverses, telles que sous forme de crèmes, de gels ou sous toute autre forme appropriée pour réaliser une teinture des fibres kératiniques, et notamment des fibres kératiniques humaines tels que les cheveux.

L'invention a aussi pour objet un dispositif à plusieurs compartiments ou "kit" de teinture comprenant au moins un premier compartiment contenant la composition tinctoriale définie ci-dessus et au moins un deuxième compartiment contenant une composition oxydante. Ce dispositif peut être équipé d'un moyen permettant de délivrer sur les cheveux le mélange souhaité, tel que les dispositifs décrits dans la demande de brevet FR-A-2 586 913.

Les exemples qui suivent servent à illustrer l'invention sans toutefois présenter un caractère limitatif.

### EXEMPLES

### Composition colorante selon l'invention

La composition suivante a été réalisée.

| **Composition colorante** | **Composition** |
|---|---|
| Cétyl hydroxyéthylcellulose (Polysurf 67 commercialisé par la société Aqualon) | 0,5 g |
| Monoéthanolamide d'acide stéarique | 4,8 g |
| PEG-40 stéarate | 1,8 g |
| p-aminophénol | 0,24 g |
| Toluène-2,5-diamine | 0,28 g |
| | - |
| m-aminophénol | 0,02 g |
| 2,4-diaminophénoxyéthanol, HCl | 0,02 g |
| 2-amino-3-hydroxypyridine | 0,2 g |
| 6-hydroxyindole | 0,01 g |
| 4-amino-2-hydroxytoluène | 0,24 g |
| 2-méthyl-5-hydroxyéthylaminophénol | 0,15 g |
| Acide oléïque | 3 g |
| Solution aqueuse à 20 % en poids en NH₃ | 5 g |
| TiO₂ | 0,3 g |
| Monoéthanolamine | 0,8 g |
| | - |
| Solution aqueuse à 40 % en poids Polyquaternium-6 (Merquat 100 commercialisé par la société Ondéo) | 1,6 g |
| Acide éthylène diamine tétraacétique (EDTA) | 0,2 g |
| Solution aqueuse à 60 % en poids de chlorure d'hexadiméthrine (Mexomère PO commercialisé par la société Chimex) | 1,2 g |
| Hydroxypropylméthylcellulose | 0,19 g |
| Oleth-30 | 1,5 g |
| Stéareth-2 | 5,5 g |
| Glycéryl lauryl éther | 0,5 g |
| Alcools en C₂₀-C₂₂ (Nafol 2022 EN commercialisé par la société Sasol) | 3 g |
| Réducteur, antioxydant | q.s. |
| Eau déminéralisée q.s.p. | 100 g |

### Protocole d'application

La composition est diluée extemporanément, avec une fois et demie son poids d'une composition oxydante de pH voisin de 3 (eau oxygénée à 20 volumes) (6 % en poids d'H₂O₂). Le mélange se fait facilement et présente une bonne viscosité ; il est appliqué facilement sur des cheveux gris, à 90 % de cheveux blancs, à raison de 10 g pour 1 g de cheveux, pendant 30 minutes. Les cheveux sont ensuite rincés, lavés avec un shampooing standard et séchés.

La coloration capillaire est évaluée de manière visuelle. Les résultats obtenus sur les cheveux naturels gris, à 90 % de cheveux blancs, après traitement, sont les suivants :

| | **Nuance** |
|---|---|
| **Composition** | Blond acajou cuivré |

La coloration possède de bonnes propriétés notamment en termes de sélectivité et d'intensité. La composition obtenue est stable dans le temps.

## Revendications

1. Composition tinctoriale pour fibres kératiniques, comprenant, dans un milieu approprié pour la teinture :
(A) un ou plusieurs dérivé(s) non ionique(s) de cellulose comprenant un ou plusieurs substituant(s) hydrophobe(s) comprenant de 8 à 30 atomes de carbone ;
(B) un ou plusieurs ester(s) d'acide gras en C8-C30 ; et
(C) un ou plusieurs colorant(s) d'oxydation,
l'ester d'acide gras (B) étant choisi parmi les monoesters, diesters et triesters issus de la réaction de monoacides ou diacides, linéaires ou ramifiés, saturés ou insaturés, comportant de 8 à 30 atomes de carbone, éventuellement hydroxylés, avec des polyols, saturés ou insaturés, linéaires, ramifiés ou cycliques, comportant de 2 à 1000 atomes de carbone et de 1 à 30 groupement(s) hydroxyle, les polyols étant choisis parmi l'éthylène glycol, le propylène glycol, le glycérol, les polyéthylène glycols et les polypropylène glycols,
l'ester d'acide gras (B) étant choisi parmi les mono- ou distéarate d'éthylène glycol, les mono- ou distéarate de polyéthylène glycols, l'isostéarate de glycéryle, le dipélargonate de propylèneglycol, le trihydroxystéarate de glycéryle.

2. Composition tinctoriale selon la revendication 1, **caractérisée en ce que** le dérivé non ionique de cellulose (A) est une hydroxyéthylcellulose substituée par un ou plusieurs substituant(s) hydrophobe(s) comprenant de 8 à 30 atomes de carbone.

3. Composition tinctoriale selon l'une quelconque des revendications précédentes, **caractérisée en ce que** le substituant hydrophobe est un groupe alkyle en C₁₀-C₂₂.

4. Composition tinctoriale selon l'une quelconque des revendications précédentes, **caractérisée en ce que** le substituant hydrophobe est un groupement cétyle.

5. Composition tinctoriale selon l'une quelconque des revendications précédentes, **caractérisée en ce que** le degré de substitution hydrophobe va de 0,1 à 10 % en poids, de préférence de 0,1 à 1 % en poids et de manière plus préférée de 0,4 à 0,8 % en poids, du poids total du polymère.

6. Composition tinctoriale selon l'une quelconque des revendications précédentes, **caractérisée en ce que** la concentration en dérivé(s) non-ionique(s) de cellulose (A) va de 0,01 à 10 % en poids, de préférence de 0,05 à 3 % en poids et de manière plus préférée de 0,1 à 1 % en poids, par rapport au poids total de la composition.

7. Composition tinctoriale selon l'une quelconque des revendications précédentes, **caractérisée en ce qu'**elle comprend en outre un ou plusieurs amide(s) d'une alcanolamine et d'un acide gras en C₁₄-C₃₀, de préférence choisi(s) parmi les amides d'une alcanolamine en C₂-C₁₀ et d'un acide gras en C₁₄-C₃₀, et plus préférentiellement parmi les amides d'une alcanolamine en C₂-C₆ et d'un acide gras en C₁₄-C₂₂.

8. Composition tinctoriale selon l'une quelconque des revendications précédentes, **caractérisée en ce que** la concentration en ester(s) d'acide gras (B) va de 0,01 à 10 % en poids, de préférence de 0,2 à 10 % en poids, et de manière plus préférée de 0,5 à 6 % en poids, par rapport au poids total de la composition.

9. Composition tinctoriale selon l'une quelconque des revendications précédentes, **caractérisée en ce que** le colorant d'oxydation (C) est choisi parmi les bases d'oxydation, les coupleurs d'oxydation, et leurs sels d'addition.

10. Composition tinctoriale selon l'une quelconque des revendications précédentes, **caractérisée en ce qu'**elle comprend au moins un agent oxydant.

11. Procédé de teinture d'oxydation des fibres kératiniques, **caractérisé en ce qu'**on applique sur les fibres une composition tinctoriale telle que définie dans l'une quelconque des revendications 1 à 9 en présence d'au moins un agent oxydant, pendant un temps suffisant pour développer la couleur désirée.

12. Dispositif à plusieurs compartiments, **caractérisé en ce qu'**il comprend au moins un premier compartiment contenant une composition tinctoriale telle que définie dans l'une quelconque des revendications 1 à 9 et au moins un deuxième compartiment contenant au moins un agent oxydant.

13. Utilisation de la composition définie dans l'une des revendications 1 à 10 pour la teinture des fibres kératiniques, en particulier, des fibres kératiniques humaines telles que les cheveux.

## Patentansprüche

1. Färbezusammensetzung für Keratinfasern, die in einem zum Färben geeigneten Medium Folgendes umfasst:
(A) ein oder mehrere nichtionische(s) Zellulosederivat(e), umfassend einen oder mehrere hydrophobe(n) Substituenten, umfassend 8 bis 30 Kohlenstoffatome;
(B) einen oder mehrere C₈-C₃₀-Fettsäureester; sowie
(C) einen oder mehrere Oxidationsfarbstoff(e), wobei der Fettsäureester (B) aus der Reihe der aus der Reaktion von linearen oder verzweigten, gesättigten oder ungesättigten, gegebenenfalls hydroxylierten Monosäuren oder Disäuren mit 8 bis 30 Kohlenstoffatomen mit gesättigten oder ungesättigten, linearen, verzweigten oder cyclischen Polyolen mit 2 bis 1000 Kohlenstoffatomen und 1 bis 30 Hydroxylgruppen erhaltenen Monoester, Diester und Triester ausgewählt ist, wobei die Polyole aus der Reihe Ethylenglykol, Propylenglykol, Glycerin, Polyethylenglykole und Polypropylenglykole ausgewählt sind,
wobei der Fettsäureester (B) aus der Reihe Ethylenglykolmonostearat oder -distearat, Polyethylenglykolmonostearat oder -distearat, Glycerylisostearat, Propylenglykoldipelargonat, Glyceryltrihydroxystearat ausgewählt ist.

2. Färbezusammensetzung nach Anspruch 1, **dadurch gekennzeichnet, dass** es sich bei dem nichtionischen Zellulosederivat (A) um eine durch einen oder mehrere hydrophobe(n) Substituenten, umfassend 8 bis 30 Kohlenstoffatome substituierte Hydroxyethylzellulose handelt.

3. Färbezusammensetzung nach einem der vorhergehenden Ansprüche, **dadurch gekennzeichnet, dass** es sich bei dem hydrophoben Substituenten um eine C₁₀-C₂₂-Alkylgruppe handelt.

4. Färbezusammensetzung nach einem der vorhergehenden Ansprüche, **dadurch gekennzeichnet, dass** es sich bei dem hydrophoben Substituenten um eine Cetylgruppe handelt.

5. Färbezusammensetzung nach einem der vorhergehenden Ansprüche, **dadurch gekennzeichnet, dass** der Grad der hydrophoben Substitution 0,1 bis 10 Gew.-%, vorzugsweise 0,1 bis 1 Gew.-% und am stärksten bevorzugt 0,4 bis 0,8 Gew.-% des Gesamtgewichts des Polymers beträgt.

6. Färbezusammensetzung nach einem der vorhergehenden Ansprüche, **dadurch gekennzeichnet, dass** die Konzentration an nichtionischem/n Zellulosederivat(en) (A) 0,01 bis 10 Gew.-%, vorzugsweise 0,05 bis 3 Gew.-% und am stärksten bevorzugt 0,1 bis 1 Gew.-% in Bezug auf das Gesamtgewicht der Zusammensetzung beträgt.

7. Färbezusammensetzung nach einem der vorhergehenden Ansprüche, **dadurch gekennzeichnet, dass** sie außerdem ein oder mehrere Amide eines Alkanolamins und einer C₁₄-C₃₀-Fettsäure, vorzugsweise ausgewählt aus den Amiden eines C₂-C₁₀-Alkanolamins und einer C₁₄-C₃₀-Fettsäure und weiter bevorzugt aus Amiden eines C₂-C₆-Alkanolamins und einer C₁₄-C₂₂-Fettsäure, umfasst.

8. Färbezusammensetzung nach einem der vorhergehenden Ansprüche, **dadurch gekennzeichnet, dass** die Konzentration an Fettsäureester(n) (B) 0,01 bis 10 Gew.-%, vorzugsweise 0,2 bis 10 Gew.-% und am stärksten bevorzugt 0,5 bis 6 Gew.-% in Bezug auf das Gesamtgewicht der Zusammensetzung beträgt.

9. Färbezusammensetzung nach einem der vorhergehenden Ansprüche, **dadurch gekennzeichnet, dass** der Oxidationsfarbstoff (C) aus der Reihe der Oxidationsbasen, der Oxidationskuppler und ihren Additionssalzen ausgewählt ist.

10. Färbezusammensetzung nach einem der vorhergehenden Ansprüche, **dadurch gekennzeichnet, dass** sie mindestens ein Oxidationsmittel umfasst.

11. Verfahren für die Oxidationsfärbung von Keratinfasern, **dadurch gekennzeichnet, dass** man eine Färbezusammensetzung wie in einem der Ansprüche 1 bis 9 definiert in Gegenwart von mindestens einem Oxidationsmittel über einen Zeitraum, der ausreicht, um die gewünschte Farbe zu entwickeln, auf die Fasern aufbringt.

12. Einheit mit mehreren Kompartimenten, **dadurch gekennzeichnet, dass** sie mindestens ein erstes Kompartiment umfasst, das eine Färbezusammensetzung wie in einem der Ansprüche 1 bis 9 definiert enthält, und mindestens ein zweites Kompartiment, das mindestens ein Oxidationsmittel enthält, umfasst.

13. Verwendung der in einem der Ansprüche 1 bis 10 definierten Zusammensetzung zum Färben von Keratinfasern, insbesondere menschlichen Keratinfasern wie dem Haar.

## Claims

1. Dye composition for keratin fibres, comprising, in a medium suitable for dyeing:
(A) one or more nonionic derivative (s) of cellulose comprising one or more hydrophobic substituent(s) containing from 8 to 30 carbon atoms;
(B) one or more C₈-C₃₀ fatty acid ester(s); and
(C) one or more oxidation dye(s), the fatty acid ester (B) being chosen from the monoesters, diesters and triesters derived from the reaction of linear or branched, saturated or unsaturated, optionally hydroxylated monoacids or diacids containing from 8 to 30 carbon atoms, with saturated or unsaturated, linear, branched or cyclic polyols comprising from 2 to 1000 carbon atoms and from 1 to 30 hydroxyl group(s), the polyols being chosen from ethylene glycol, propylene glycol, glycerol, polyethylene glycols and polypropylene glycols,
the fatty acid ester (B) being chosen from ethylene glycol monostearate or distearate, polyethylene glycol monostearate or distearate, glyceryl isostearate, propylene glycol dipelargonate, and glyceryl trihydroxystearate.

2. Dye composition according to Claim 1, **characterized in that** the nonionic derivative of cellulose (A) is a hydroxyethylcellulose substituted with one or more hydrophobic substituent(s) containing from 8 to 30 carbon atoms.

3. Dye composition according to either one of the preceding claims, **characterized in that** the hydrophobic substituent is a C₁₀-C₂₂ alkyl group.

4. Dye composition according to any one of the preceding claims, **characterized in that** the hydrophobic substituent is a cetyl group.

5. Dye composition according to any one of the preceding claims, **characterized in that** the degree of hydrophobic substitution ranges from 0.1% to 10% by weight, preferably from 0.1% to 1% by weight, and more preferably from 0.4% to 0.8% by weight, of the total weight of the polymer.

6. Dye composition according to any one of the preceding claims, **characterized in that** the concentration of nonionic derivative(s) of cellulose (A) ranges from 0.01% to 10% by weight, preferably from 0.05% to 3% by weight, and more preferably from 0.1% to 1% by weight, relative to the total weight of the composition.

7. Dye composition according to any one of the preceding claims, **characterized in that** it also comprises one or more amide (s) of an alkanolamine and of a C₁₄-C₃₀ fatty acid, preferably chosen from the amides of a C₂-C₁₀ alkanolamine and of a C₁₄-C₃₀ fatty acid, and more preferably from the amides of a C₂-C₆ alkanolamine and of a C₁₄-C₂₂ fatty acid.

8. Dye composition according to any one of the preceding claims, **characterized in that** the concentration of fatty acid ester(s) (B) ranges from 0.01% to 10% by weight, preferably from 0.2% to 10% by weight, and more preferably from 0.5% to 6% by weight, relative to the total weight of the composition.

9. Dye composition according to any one of the preceding claims, **characterized in that** the oxidation dye (C) is chosen from oxidation bases and oxidation couplers, and addition salts thereof.

10. Dye composition according to any one of the preceding claims, **characterized in that** it comprises at least an oxidizing agent.

11. Process for the oxidation dyeing of keratin fibres, **characterized in that** a dye composition as defined in any one of Claims 1 to 9 is applied to the fibres, in the presence of at least one oxidizing agent for a period of time sufficient to develop the desired colour.

12. Multicompartment device, **characterized in that** it comprises at least a first compartment containing a dye composition as defined in any one of Claims 1 to 9 and at least a second compartment containing at least one oxidizing agent.

13. Use of the composition defined in any one of Claims 1 to 10, for dyeing keratin fibres, in particular human keratin fibres such as the hair.
